# EUROPÄISCHE PATENTANMELDUNG

(11) **EP 2 181 699 A1**
(43) Veröffentlichungstag der Anmeldung: **05.05.2010**
(21) Anmeldenummer: 09013270.5
(22) Anmeldetag: 21.10.2009
(51) Int. Cl.: A61K 8/894, A61Q 17/04, A61Q 17/00

(54) **Kosmetische Formulierung mit Siloxanelastomeren zur Verbesserung der mikrobiologischen Stabilität**

(30) Priorität: 22.10.2008 DE 102008053788
(71) Anmelder: Beiersdorf AG, 20245 Hamburg (DE)
(72) Erfinder: Storbeck, Celina, 25474 Bönningstedt (DE); Skubsch, Kerstin, 25497 Prisdorf (DE); Köhler, Manuela, Dr., 20144 Hamburg (DE)
(74) Vertreter: Hartmann, Jost

(57) **Zusammenfassung**

Die Erfindung beschreibt die Verwendung von ABC-Siloxanelastomere zur Verbesserung der mikrobiologische Stabilität kosmetischer Zubereitungen.

## Beschreibung

Die Erfindung beschreibt kosmetische Zubereitungen umfassend ABC-Siloxanelastomere zur Verbesserung der mikrobiologischen Stabilität.

Siloxanelastomere werden in kosmetischen Zubereitungen aufgrund ihrer seidigen und pudrigen Sensorikeigenschaften eingesetzt. Ist man in kosmetischen Formulierungen aufgrund der gewünschten Technologie auf polare Subtanzen angewiesen, so sind Siloxanelastomere, die als Gel in unpolaren Ölen, in der Regel Silikonölen, oft nicht verwendbar, da sie Instabilitäten provozieren. Aus diesem Grunde werden deswegen oft Stärken oder andere Puderrohstoffe eingesetzt. Gleichzeitig ist bekannt, dass Puderrohstoffe aller Art, insbesondere natürlicher Herkunft, die mikrobiologische Stabilität von kosmetischen Formulierungen negativ beeinflussen. Somit ist der gesamte Formulierungsfreiraum eingeschränkt.

Nachteil bei den bisher bekannten Elastomeren ist deren Einschränkung auf Silikonöle bzw. auf zwingend flüchtige Silikonöle und dass die Verarbeitung in Kosmetika nur mit flüchtigem Silikonöl funktioniert. Silikonöle sind per se unpolar.

DE 102005019548, WO 2005094775 und EP 1391193 beschreiben bekannte Siloxanelastomere wie Dimethicon/Vinyl Dimethicon Crosspolymer oder Dimethicon/Polysilicon 11.

In den WO 2007109260, WO 2007109240 und WO 2008085360 werden neuartige Siloxanelastomere beschrieben. Diese Silioxanelastomere, genauer Siloxanpolyetherelastomere, erhält man durch die Reaktion eines Organosiloxans mit einem, mindestens zwei SiH Gruppen enthaltendem Cyclosiloxan und einer Substanz oder mehreren Substanzen, mit mindestens 2 aliphatischen ungesättigten Gruppen im Molekül unter Verwendung eines Hydrosilylierungskatalysators. Diese in den WO 2007109260, WO 2007109240 und WO 2008/085360 beschriebenen Siloxanelastomere in Gelform können mit Körperpflege- oder Gesundheitsstoffen, die pharmakologische Wirkungen zeigen, wie Vitamine oder Hormone, kombiniert werden. Diese in WO 2007109260, WO 2007109240 und WO 2008/085360 beschriebenen Siloxanelastomere können auch in nicht flüchtigen, polaren Ölen zur Verfügung gestellt werden.

Aufgrund ihrer Inhaltsstoffe sind Kosmetika durch eine mikrobielle Kontamination gefährdet. Bei nicht ausreichend geschützten Produkten kann es daher zu einer gesundheitlichen Gefährdung des Verwenders bzw. zum Verderb des Produktes kommen. Letzteres kann sich durch unangenehmen Geruch, Verfärbung, Schimmelbildung oder auch durch eine physikalische Instabilität zeigen.

Eine große Vielfalt von Mikroorganismen führt zu Verunreinigungen in Kosmetika. In der Mehrzahl handelt es sich hierbei um Bakterien, geringer ist der Anteil an Hefen und Schimmelpilzen. Eine Kontamination von Kosmetika ist zum einen während der gesamten Herstellung, zum anderen während der Benutzung möglich. Im letzteren Fall, insbesondere bei Produkten, welche in Tiegel angeboten werden, da hier der Verbraucher in die Masse fasst und sie dabei potentiell mit Mikroben kontaminieren kann. Aus diesem Grund werden bestimmte Produkte nur in Tuben oder aus Spendern angeboten. Diese Verpackungsformen werden jedoch zum Teil als weniger attraktiv und kosmetisch empfunden.

Generell müssen alle Kosmetika neben der physikalischen Stabilität auch eine mikrobiologische Stabilität ausweisen. Diese Stabilität wird im Allgemeinen durch einen mehrwöchigen Keimbelastungstest nachgewiesen.

Das Gefühl eines Produktes während der Applikation beeinflußt das Erleben des Produktes immens. Aus diesem Grunde ist man bemüht für jedes Produkt das Gefühl während der Applikation optimal abzustimmen. Oft gewünschte Eigenschaften der Kosmetika sind seidig, pudrig, samtig. Um dieses Gefühl zu erzeugen werden verschiedene Rohstoffe eingesetzt. Besonders effektiv sind dabei Siloxanelastomere. Da bekannte Siloxanelastomere immer gequollen in flüchtigen, unpolaren Silikonölen bereitgestellt werden, ist man bei einigen Technologien gezwungen alternative Rohstoffe zu verwenden. Hier werden oft Puderrohstoffe verwendet, die Derivate von natürlichen Substanzen, insbesondere Kohlenhydraten, wie Stärken, Cellulosen, etc. verwendet, da man sie gut in polaren Formulierungen formulieren kann. Es liegt aber auf der Hand, das solche Substanzen ideale Nahrung für Mikoben wie Bakterien, Hefen und Pilze darstellen. Um die mikrobiologische Stabilität zu gewährleiten, muss man dann zu höheren Mengen an Konservierungsmittel oder zu wirkungsstärkeren jedoch beim Konsumenten ungern gesehenen Substanzen, wie halogenierten organischen Substanzen, FAL-Abspaltern etc. greifen.

Aufgabe ist es daher kosmetische Zubereitungen bereit zu stellen, welche statt der üblichen, mikrobiologisch bedenklichen Stärkederivate Siloxaneelastomere enthalten, ohne die physikalische Stabilität der Formel zu beeinflussen und dennoch eine ausreichende mikrobiologische Stabilität aufweisen.

Bedeutsam wäre es aber darüber hinaus für den kosmetischen Einsatz stabile und vielfältig einsetzbare kosmetische Zubereitungen bereit zu stellen.

Gelöst werden diese Aufgaben durch kosmetische Formulierungen umfassend ABC-Siloxanelastomere entsprechend Anspruch 1.

Die erfindungsgemäße kosmetische Formulierung umfasst ein oder mehrere ABC-Siloxanelastomere, erhältlich aus der Reaktion von
A-Organohydrogensiloxanen mit mindestens zwei SiH tragenden - Cyclosiloxanringen pro Molekül mit
B-Stoffen oder Mischungen mit mindestens zwei aliphatischen ungesättigten Gruppen oder Hydrocarbongruppen pro Molekül und einem
C-Hydrosilylationskatalysator.

Bevorzugt werden die ABC-Siloxanelastomere mit polaren Ölen oder Wachsen in der Zubereitung kombiniert.

Insbesondere Organohydrogensiloxane A der Struktur: 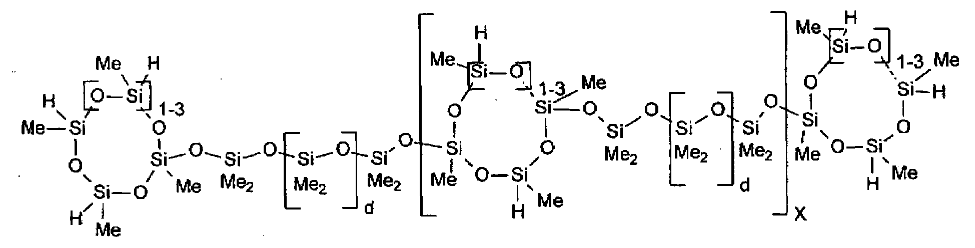 und/oder 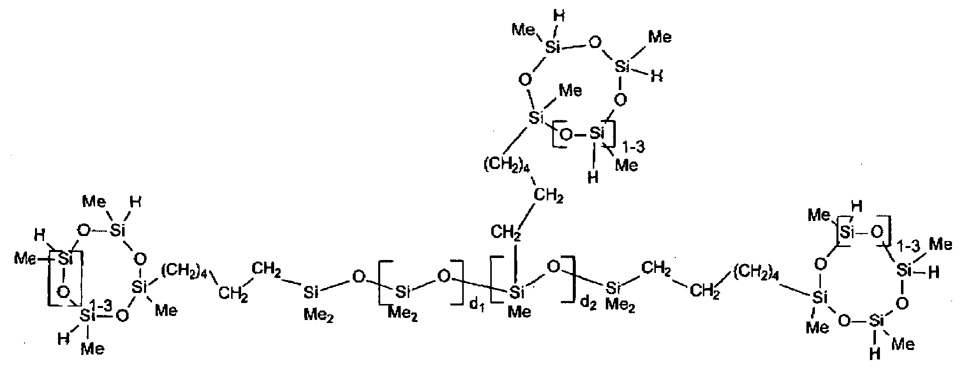 mit x, d, d1 und d2 im Bereich von 0 bis 11.000 sind erfindungsgemäß bevorzugt zu verwenden.

Erfindungsgemäße ABC-Siloxanelastomere sind vor allem die in den Druckschriften WO 2007109260, WO 2007109240 und WO 2008/085360 beschriebenen Silikon Elastomere, Silikon Polyether Elastomere und deren Gelformen. Es wird hiermit ausdrücklich zu den in der Druckschriften WO 2007109260, WO 2007109240 und WO 2008/085360 dargestellten Elastomeren und deren Herstellung Bezug genommen.
Bevorzugte Beispiele der erfindungsgemäßen Siloxanelastomere sind Dimethicone/PPG-20 Crosspolymer, Dimethicone/ Bis-Isobutyl PPG-20 Crosspolymer bzw. Bis-Vinyl Dimethicone/PPG-20 Crosspolymer.

Diese erfindungsgemäßen neuartigen ABC-Siloxanelastomere unterscheiden sich von den klassischen bekannten Siloxanelastomeren, wie das Dimethicon/Vinyl Dimethicon Crosspolymer, aufgrund einer unterschiedlichen chemischen Struktur und bedingen dadurch auch Unterschiede in ihren Eigenschaften.

Überraschenderweise wurde nun gefunden, dass durch den Einsatz der ABC-Siloxanelastomere die mikrobiologische Stabilität erhöht werden konnte. Kosmetische Formulierungen umfassend die ABC-Siloxanelastomere anstelle der sonst verwendeten Puderrohstoffe oder anderen Elastomeren führen erstaunlicherweise zu einer besseren Absterbekinetik bezüglich Bakterien, Hefen und Pilzen innerhalb eines Testzeitraumes von 28 Tagen. Produkte die sonst den Keimbelastungstest (KBT) nicht bestanden haben, haben überraschenderweise durch den Austausch von Puderrohstoffen durch ABC-Siloxanelastomere bei sonst gleicher Formulierung den KBT bestanden und sind physikalisch stabil. Das heißt auch, dass bei Produkten durch den Austausch von Puderrohstoffen durch ABC-Siloxanelastomere die sonst nicht reduzierbare Menge der Konservierungsmittel/ Konservierungsmittelhelfer reduziert werden kann, ohne dass die mikrobiologische und physikalische Stabilität beeinträchtigt wird.

Die Verwendung von ein oder mehreren ABC-Siloxanelastomere, erhältlich aus der Reaktion von
A-Organohydrogensiloxanen mit mindestens zwei SiH tragenden Cyclosiloxanringen pro Molekül, mit
B-Stoffen oder Mischungen mit mindestens zwei aliphatischen ungesättigten Gruppen oder Hydrocarbongruppen pro Molekül und einem
C-Hydrosilylationskatalysator,
   in kosmetischen Zubereitungen führt zur Verbesserung der mikrobiologische Stabilität der Zubereitung.
   Erfindungsgemäß ist daher deren entsprechende Verwendung zur Verbesserung der mikrobiologische Stabilität kosmetischer Zubereitungen.

Der Zubereitung werden die ABC-Siloxanelastomere vorteilhaft in Kombination mit ein oder mehereren polaren Öle und Wachsen zugesetzt.
Bevorzugte Anteile der ABC-Siloxanelastomere liegen im Bereich von 1 bis 25 Gew.%, bezogen auf die Gesamtmasse der Zubereitung, insbesondere im Bereich von 2 bis 10 Gew.%.
In Vergleichsuntersuchungen wurden folgende Beispielzubereitungen 23 bis 26 hinsichtlich der mikrobiellen Stabilität getestet.

| | | | | |
|---|---|---|---|---|
| **Inhaltsstoffe/Beispiele** | **23** | **24** | **25** | **26** |
| Glyceryl Stearate | 2,6 | 2,6 | 2,6 | 2,6 |
| Caprylic/Capric Triglyceride | 2,5 | 2,5 | 2,5 | 2,5 |
| Cetearyl Alcohol | 3 | 3 | 3 | 3 |
| PEG-40 Stearate | 0,8 | 0,8 | 0,8 | 0,8 |
| Aqua + Trisodium EDTA | 1 | 1 | 1 | 1 |
| Ethylhexyl Methoxycinnamate + BHT | 3 | 3 | 3 | 3 |
| Dimethicone | 0,35 | 0,35 | 0,35 | 0,35 |
| Cyclomethicone | 2,15 | 2,15 | 2,15 | 2,15 |
| C12-15 Alkyl Benzoate | 2,5 | 2,5 | 2,5 | 2,5 |
| Butyl Methoxydibenzoylmethane | 1 | 1 | 1 | 1 |
| Nylon-12 | 1,5 | 1,5 | | 1,5 |
| Glycerin | 8,65 | 8,65 | 8.65 | 8,65 |
| Aqua + Sodium Hydroxide | 0,054 | 0,054 | 0,054 | 0,054 |
| Carbomer | 0,1 | 0,1 | 0,1 | 0,1 |
| Parfum | 0,25 | 0,25 | 0,25 | 0,25 |
| Tapioca Starch + Aqua | 1,5 | | 1,5 | 1,5 |
| Dicaprylyl Carbonate | 2,5 | 2,5 | 2,5 | 2,5 |
| Methylpropanediol | 4 | 4 | 4 | 4 |
| Benzethonium Chloride | 0,05 | 0,05 | 0,05 | 0,05 |
| Dimethicone/ Bis-Isobutyl PPG-20 Crosspolymer | 0 | 1,5 | 1,5 | 3 |
| Aqua | Ad | Ad | Ad | Ad |

Die Beispielzubereitungen wurden in einen mikrobiologischen Vollbelastungstest gegeben. Es wurden Keimbelastungstests mit den Keimen Staphylococcus aureus/epidermis, Pseudomonas aeruginosa,Pseudomonas putida/cepacia, Escherichia Coli/Enterob. Gergoviae, Aspergillus Brasiliensis und Candida albicans/parapsilosis/guilliermondii durchgeführt. Es wurde insbesondere gegen die Keime Pseudomonas putida/cepacia getestet (KBE - Koloniebildende Einheit).

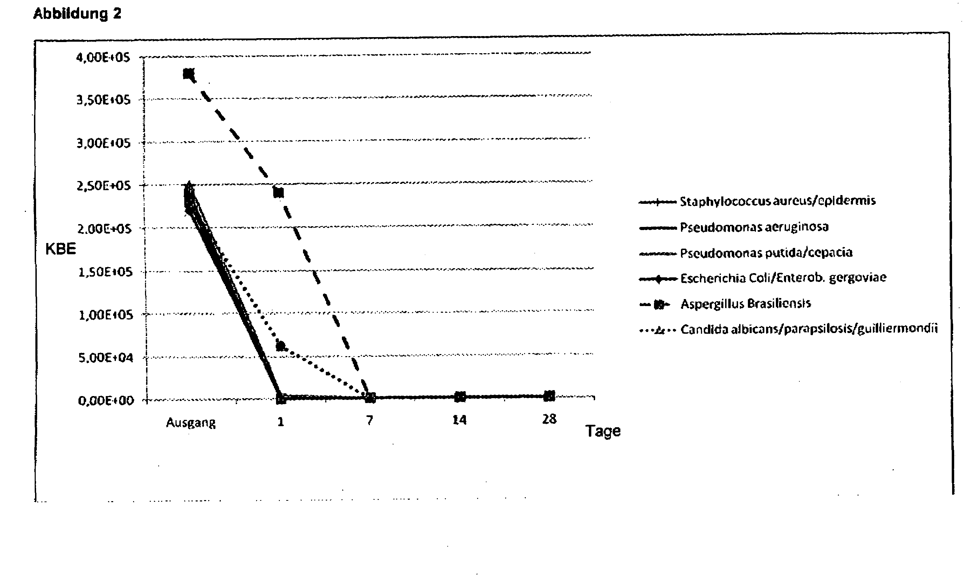

Abbildung 2 zeigt die keimabtötende Wirkung der erfindungsgemäßen Zubereitung (Beispiel 26) aller relevanten Keime.
Häufig scheitert das Bestehen einer Keimbelastung daran, das nicht alle Keime schnell genug absterben. Pseudomonas putida/cepacia stellen häufig derartige Keime dar, die zu einem Nichtbestehen des Belastungstests führen.
So zeigt sich im Vergleich der Zubereitungen mit und ohne erfindungsgemäßen Siloxanelastomeren, dass diese Keime nur durch die erfindungsgemäßen Zubereitungen vorteilhaft verringert werden.
Erst durch die Zugabe des Elastomers sind auch Pseudomonas putidalcepacia genügend von der Formel (Beispiel 24, 25und insbesondere 26) reduziert worden und erst so konnte der Keimbelastungstest bestanden werden.

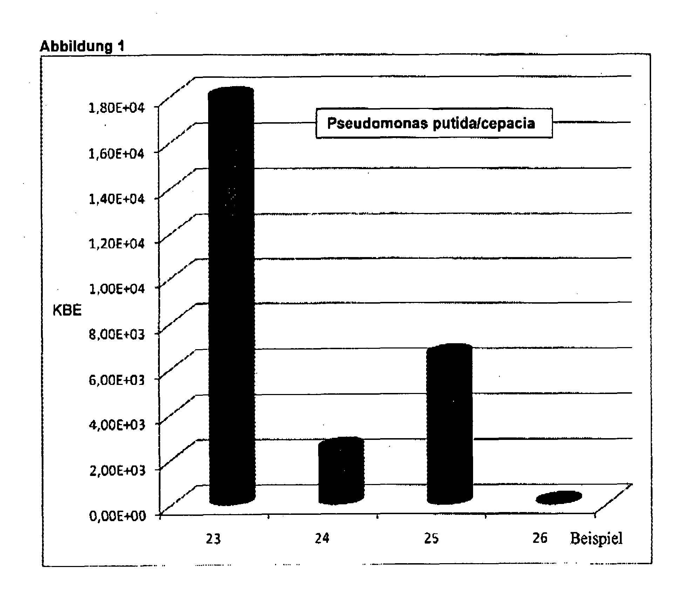

Abbildung 1 zeigt den Rückgang der Pseudomonas putida/cepacia Keime nach 7 Tagen. Ohne Zusatz an erfindungsgemäßen ABC Siloxanelastomeren (Beispiel 23) ist die mikrobielle Stabilität ungenügend, hingegen wird diese Stabilität durch den Zusatz an den ABC Siloxanelastomeren verbessert, wie die Beispiele 24, 25 und 26 zeigen.
Deutlich wird anhand der Untersuchungen auch, dass wenn Puderrohstoffe wie Tapioca Starch und/oder Nylon-12 in den Zubereitungen enthalten sind, die Konservierung schwieriger wird. Der Zusatz an erfindungsgemäßen Elastomeren diese Schwierigkeiten jedoch vermindern hilft.
Vorteilhaft hinsichtlich der mikrobiellen Stabilität der kosmetischen Zubereitungen zeigt sich weiterhin, dass der Anteil an ABC-Siloxanelastomeren vorzugsweise über 1,5 Gew.%, bezogen auf die Gesamtmasse der Zubereitung liegt (siehe Beispiele 24, 25 und 26).

Der Zusatz an polaren Öle oder Wachse in Kombination mit ABC-Siloxanelastomeren führt zur Stabilitätsverbesserung kosmetischer Zubereitungen und deren Kombination ist demnach bevorzugt zur Stabilitätsverbesserung kosmetischer Zubereitungen zu verwenden.

So zeigen die ABC-Siloxanelastomere mit polaren Ölen, also insbesondere anderen Ölen als Silikonölen, eine verbesserte Kompatibilität und ermöglichen so eine vielfältigere Sensorik, bessere Stabilisierung und Freisetzung von Wirkstoffen, insbesondere partikulären Stoffen.

Aus der verbesserten Kompatibilität mit verschiedenen, auch polaren Ölen im Gegensatz zu herkömmlichen Siloxanelastomeren kann erfindungsgemäß auf unpolare und insbesondere flüchtige Öle (Kohlenwasserstoffe, Silikonöle) in kosmetischen Formeln zukünftig verzichtet bzw. deren Menge reduziert werden, was einen erheblichen Formulierungsvorteil und -freiraum bietet.
Auf die in kosmetischen Zubereitungen umfassend Siloxanelastomeren ansonsten enthaltenen Silikonöle, insbesondere flüchtigen Silikonöle, kann erfindungsgemäß verzichtet werden. Vorteilhaft sind die erfindungsgemäßen Zubereitungen daher silikonölfrei. Frei hießt dabei ein Anteil von weniger als 1 Gew.%, bezogen auf die Gesamtmasse der Zubereitung, bevorzugt weniger als 0,1 Gew.% an Silikonölen.

Öle und Fette unterscheiden sich unter anderem in ihrer Polarität. Es wurde vorgeschlagen, die Grenzflächenspannung gegenüber Wasser als Maß für den Polaritätsindex eines Öls bzw. einer Ölphase anzunehmen. Dabei gilt, dass die Polarität der betreffenden Ölphase umso größer ist, je niedriger die Grenzflächenspannung zwischen dieser Ölphase und Wasser ist. Erfindungsgemäß wird die Grenzflächenspannung als ein mögliches Maß für die Polarität einer gegebenen Ölkomponente angesehen.

Die Grenzflächenspannung ist diejenige Kraft, die an einer gedachten, in der Grenzfläche zwischen zwei Phasen befindlichen Linie der Länge von einem Meter wirkt. Die physikalische Einheit für diese Grenzflächenspannung errechnet sich klassisch nach der Beziehung Kraft/Länge und wird gewöhnlich in mN/m (Millinewton geteilt durch Meter) wiedergegeben. Sie hat positives Vorzeichen, wenn sie das Bestreben hat, die Grenzfläche zu verkleinern. Im umgekehrten Falle hat sie negatives Vorzeichen. Als polar werden Lipide angesehen, deren Grenzflächenspannung gegen Wasser weniger als 20 mN/m beträgt, als unpolar solche, deren Grenzflächenspannung gegen Wasser mehr als 30 mN/m beträgt. Lipide mit einer Grenzflächenspannung gegen Wasser zwischen 20 und 30 mN/m werden im Allgemeinen als mittelpolar bezeichnet.

Erfindungsgemäß ist es nun möglich Lipide mit einer Grenzflächenspannung kleiner 30 mN/m in Kombination mit den ABC-Siloxanelastomeren in kosmetischen Zubereitungen einzusetzen.

Auf den Zusatz unpolarer Lipide mit einer Grenzflächenspannung größer 30 mN/m kann hingegen verzichtet werden bzw. deren Gehalt erheblich reduziert werden.

Beispielsweise sind polare Ole, wozu auch flüssige UV-Filter zählen, notwendig um eine ausreichende Menge schwerlöslicher UV-Filter zu lösen, die wiederum nötig sind, um höhere Lichtschutzleistungen zu erreichen.

Der Verbraucher bevorzugt immer höhere Lichtschutzleistungen, um sich vor der Sonne zu schützen, möchte aber auf ein angenehmes Hautgefühl beim Auftragen solcher Formulierungen nicht verzichten. Herkömmliche Siloxanelastomere sind normalerweise nicht mit polaren Ölen kompatibel, beeinflussen allerdings das Hautgefühl positiv. Mit den ABC-Siloxanelastomeren ist es erstmals möglich, hohe Mengen an ansonsten schwerlöslichen UV-Filtern einzuarbeiten und nicht auf ein angenehmes Hautgefühl zu verzichten. Die Löslichkeit der schwerlösliche UV-Filter ist erhöht, wenn das erfindungsgemäße ABC-Siloxanelastomer eingesetzt wird.
Somit ist auch erstmals erhöhter Lichtschutz (SPF) und gleichzeitig erhöhter UV-A Schutz mit den erfindungsgemäßen Formulierungen möglich.

Zusätzlich wird durch die neuartigen Elastomere ein zusätzlicher SPF und UV-A Boost gewährleistet, d.h. die Lichtschutzleistung wird insgesamt erhöht gegenüber Formeln, die das neuartige Elastomer nicht enthalten.

Vorteilhafte polare Ölkomponenten können im Sinne der vorliegenden Erfindung gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können vorteilhaft gewählt werden aus der Gruppe Phenethylbenzoat, 2-Phenylethylbenzoat, Propylheptyl Caprylate, Isopropyl Lauroyl Sarkosinat, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.
Ferner können vorteilhaft gewählt werden Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche. Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid zu wählen. Es ist insbesondere vorteilhaft, wenn die Ölphase der erfindungsgemäßen Formulierungen einen Gehalt an C₁₂₋₁₃-Alkylbenzoat aufweist oder vollständig aus diesem besteht. Weitere Ölkomponenten können sein: Caprylic/Capric Triglyceride, Octyldodecanol, C12-15 Alkyl Benzoate, Butylenglycol Dicaprylat/Dicaprat, Dicaprylyl Carbonat, Isopropyl Palmitate, Ethylhexyl Cocoate, Cera Microcristallina + Paraffinum Liquidum, Butyrospermum Parkii, Dicaprylyl Ether, Hydrogenated Coco-Glycerides, Simmondsia Chinensis Oil, Tridecyl Stearate, Tridecyl Trimellitate, Dipentaerythrityl Hexacaprylate/Hexacaprate, Lanolin Alkohol.

Insbesondere vorteilhaft sind Lipide (Öle) mit einer Grenzflächenspannung gegen Wasser im Bereich von 20 bis 29,9 mN/m. Insbesondere werden die polaren Lipide gewählt aus der Gruppe C₁₂₋₁₅Alkylbenzoat; Butylenglycol Dicaprylat/Dicaprat, Dicaprylyl Carbonat, Isodecyl Neopentanoate und Caprylic/Capric Triglyceride,

Ebenfalls ist die Flüchtigkeit der Öle ein Charakteristikum zu dessen Einsatzmöglichkeit. Erfindungsgemäß kann auf den Zusatz bei Raumtemperatur flüchtigen Ölen ebenso verzichtet werden bzw. deren Gehalt vermindert werden.

Die erfindungsgemäßen Zusammensetzungen können ferner gegebenenfalls in der Kosmetik übliche Zusatzstoffe, beispielsweise Parfüm, Verdicker, Desodorantien, antimikrobielle Stoffe, fungizide Stoffe, bakteriozide Stoffe, UV-Filter, selbstbräunende Agenzien, rückfettende Agentien, Komplexierungs- und Sequestrierungsagentien, Perlglanzagentien, Pflanzenextrakte, Vitamine, Wirkstoffe, Konservierungsmittel, Bakterizide, Farbstoffe, Pigmente, die eine färbende Wirkung haben, Verdickungsmittel, anfeuchtende und/oder feuchthaltende Substanzen, Fette, Öle, Wachse, sofern nicht ausgeschlossen, oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate enthalten.
Moisturizer können vorteilhaft auch als Antifaltenwirkstoffe zum Schutz vor Hautveränderungen, wie sie z. B. bei der Hautalterung auftreten, verwendet werden.

Vorteilhaft sind den kosmetischen Zubereitungen Konservierungsmittel zugesetzt, deren Anteil jedoch aufgrund der ABC-Siloxanelastomere verringert werden kann. Vorteilhaft zeigt sich der Zusatz an Methylpropanediol und/oder Benzethonium Chloride. Der Anteil an Konservierungshelfern, insbesondere Methylpropanediol und/oder Benzethonium Chloride, beträgt vorteilhaft weniger als 5 Gew.%, insbesondere weniger als 4,5 Gew.% an Methylpropanediol und insbesondere weniger als 0,1 Gew.% an Benzethonium Chloride, jeweils bezogen auf die Gesamtmasse der Zubereitung. Die Verwendung der ABC-Siloxanelastomere in Kombination mit Methylpropanediol und/oder Benzethonium Chloride zur Verbesserung der mikrobiellen Stabilität kosmetischer Zubereitungen ist besonders bevorzugt, insbesondere mit den verringerten Anteilen.

Nachfolgende Beispiele zeigen bevorzugte Ausführungsformen der erfindungemäßen Formulierungen. Soweit nichts anderes angegeben beziehen sich die Zahlenwerte auf Gewichtsanteile, bezogen auf die Gesamtmasse der Formulierungen.

| **Beispiel Emulsion** | **1** | **2** | **3** | **4** | **5** |
|---|---|---|---|---|---|
| Glyceryl Stearat Citrat | 2 | 2 | 2 | | |
| Glyceryl Stearat SE | | | | 1 | 1 |
| Cetearyl Alkohol + PEG-40 Rizinusöl + Natrium Cetearyl Sulfat | | | | 2,5 | 2,5 |
| Cetearylalkohol | | | 1,5 | 1 | |
| Stearylalkohol | 2 | 1,5 | | | |
| Acrylates/C₁₀₋₃₀ Alkyl Acrylat Crosspolymer | | 0,2 | | | 0,1 |
| Xanthan Gum | 0,4 | | 0,2 | 0,2 | 0,3 |
| C₁₂₋₁₅ Alkyl Benzoat | | 3 | | | 5 |
| Dicaprylyl Carbonat | | 2 | | | |
| Myristylmyristat | | | 2 | | 1 |
| Butylenglycol Dicaprylat/Dicaprat | | | 3 | | 3 |
| Propylheptyl Caprylat | 5 | | 5 | 2 | |
| Dicaprylyl Ether | | | | | 2 |
| Octyldodecanol | 1 | | | | |
| Cyclisches Silikon | | 5 | 5 | 1 | 10 |
| Dimethicon / PPG-20 Crosspolymer und Isodecyl Neopentanoat | 10 | | | 2,25 | 12,5 |
| Dimethicon / PPG-20 Crosspolymer und Isododecan | | 5 | 10 | | |
| Lineares Silikon | 6 | 5 | | 5 | |
| 1-Methyl-1,3-propandiol | 5 | 8 | | | |
| Glycerin | 3 | 5 | 3 | 5 | 3 |
| Octan-1,2-diol | 2 | | 1 | | |
| 2-Methyl-1,3 propandiol | | | | 2 | |
| Bis-Vinyl Dimethicon / PPG-20 Crosspolymer | | 4 | 1 | 0,25 | |
| 2-(4'-Diethylamino-2'-hydoxybenzoyl)-benzoesäurehexylester | 3 | 5 | 1 | 0,5 | 2 |
| Octocrylen | | | 5 | | |
| Titandioxid | | 0,5 | 1 | | 2 |
| Phenylbenzimidazol Sulfonsäure | | | | 4 | 2 |
| Octylsalicylat | | | 5 | | |
| Polysilicon-15 | | | | | 2 |
| Ethylhexylmethoxycinnamat | | 10 | | | |
| Methylen Bis-Benzotriazolyl Tetramethylbutylphenol | 3 | | | 2 | |
| Ethylhexyltriazin | 3 2 | | | | 3 |
| Bis-Ethylhexylphenol Methoxyphenyl Triazin | | 2 | | | |
| PVP/Hexadecen Copolymer | 0,2 | 0,5 | 0,1 | 0,2 | |
| Vitamin E Acetat | 0,2 | 0,2 | 0,2 | 0,3 | 0,1 |
| Na₂H₂EDTA | 0,1 | 0,1 | 0,2 | 0,2 | 0,2 |
| Parfüm | 0,2 | 0,3 | 0,3 | 0,4 | 0,25 |
| Konservierungsmittel | 0,3 | 0,5 | 1 | 0,4 | 0,6 |
| Natriumhydroxid | q.s. | q.s. | q.s. | q.s. | q.s. |
| Wasser | ad 100,0 | ad 100,0 | ad 100,0 | ad 100,0 | ad 100,0 |

Es bilden sich bei allen Beispielen stabile Emulsionen.

| Beispiel | 6 | 7 | 8 | 9 | 10 | 11 |
|---|---|---|---|---|---|---|
| Glyceryl Stearat SE | 1 | 2 | | | | |
| Cetearyl Alkohol + PEG-40 Rizinusöl+ Natrium Cetearyl Sulfat | 2 | 1,5 | | | | |
| Natrium Stearoylglutamat | | | 0,2 | 0,4 | | |
| Sucrosepolystearat | | | 1 | 0,8 | | |
| Polyglyceryl-3 Methylglycose Distearat | | | | | 3 | 2,5 |
| Sorbitanstearat | | | | | 1 | 0,8 |
| Copolymer aus Vinylpyrrolidon und Acrylsäure | | | 1.5 | | | |
| Stearylalkohol | | | | | 2 | |
| Cetylalkohol | 2 | 2 | | | | 2 |
| Acrylates/C₁₀₋₃₀ Alkyl Acrylat Crosspolymer | | | | 0,3 | 0,2 | 0,1 |
| Carbomer | | | | 0,2 | | 0,1 |
| Xanthan Gum | 0,5 | 0,3 | | | | |
| C₁₂₋₁₅ Alkyl Benzoat | | 3 | 5 | | | |
| Butylenglycol Dicaprylat/Dicaprat | | | | | | |
| Dicaprylyl Carbonat | 4 | | 2 | | 2 | |
| Octyldodecanol | | | | | | 2 |
| Cyclisches Silikon | 3 | | 2 | 10 | 5 | |
| Dimethicon / PPG-20 Crosspolymer und Isodecyl Neopentanoat | 25 | | 18 | 5 | | 4,3 |
| Bis-Vinyl Dimethicon/PPG-20 Crosspolymer und Isododecan | | 12,25 | 10 | 5 | 15 | 3,5 |
| Lineares Silikon | 2 | 5 | 8 | 5 | | |
| Glycerin | 1 | 2 | 4 | 5 | 2 | 8 |
| Ethanol | 4 | 3 | 4 | | | 4 |
| 2-(4'-Diethylamino-2'-hydoxybenzoyl)-benzoesäurehexylester | 2 | | 1,5 | | 0,5 | 6 |
| Octacrylen | | | | | | 7,5 |
| Butyl Methoxydibenzoylmethan | | 2,5 | | 3 | 2 | |
| Ethylhexylmethoxycinnamat | 4 | | | | | 7,5 |
| Octylsalicylat | | | 5 | | | |
| Phenylbenzimidazol Sulfonsäure | 2 | 1 | 1 | | | |
| Titandioxid | | | | 5 | | 3 |
| Bis-Ethylhexylphenol Methoxyphenyl Triazin | | | 2 | | | 2 |
| Isoamylmethoxycinnamat | | 5 | | | | |
| Ethylhexyltriazin | | | | | 2 | 2 |
| Vitamin E Acetat | 0,1 | 0,2 | 0,1 | 0,5 | 0,25 | 0,3 |
| Na2H2EDTA | 0,05 | 0,2 | 0,2 | 0,2 | 0,2 | 0,5 |
| Stärke | 2 | 1 | | | 3 | |
| Parfüm | | | 0,2 | 0,1 | 0,3 | 0,25 |
| Konservierungsmittel | q.s. | q.s. | q.s. | q.s. | q.s. | q.s. |
| Natriumhydroxid, Farbstoffe | q.s. | q.s. | q.s. | q.s. | q.s. | q.s. |
| Wasser | ad 100,0 | ad 100,0 | ad 100,0 | ad 100,0 | ad 100,0 | ad 100,0 |

| Beispiel | 12 | 13 | 14 | 15 | 16 | 17 |
|---|---|---|---|---|---|---|
| Glycerylstearat | 2,5 | 2 | 1,2 | 1 | | |
| PEG-40 Stearat | 1 | | | | | |
| PEG-100 Stearat | | 2,5 | | | | |
| Ceteareth-20 | | | | | 1 | |
| Stearinsäure | | | 2,5 | 3 | | |
| Cetearylalkohol | 4 | | | 2 | | |
| Stearylalkohol | | 2 | 1 | | | |
| Cetylalkohol | | | 1 | 1 | | |
| Acrytates/C₁₀₋₃₀ Alkyl Acrylat Crosspolymer | | | 0,05 | 0,2 | 0,2 | 0,5 |
| Carbomer | 0,1 | | 0,2 | | | |
| Xanthan Gum | | 0,3 | | | | |
| Triheptanoin | | | 2 | | | |
| C₁₂₋₁₅ Alkyl Benzoat | | 7 | | | 3 | 3 |
| Butylenglycol Dicaprylat/Dicaprat | 2 | 4 | | | 5 | |
| Dicaprylyl Carbonat | 4 | | | | | 2 |
| Phenylethylbenzoat | | | 5 | 5 | 4 | |
| Diisopropytsebacat | | 3 | | 5 | | |
| Cyclisches Silikon | 3 | | | | | |
| 2-Propylheptyloctanoat | 4 | | 3 | | 2 | 7,5 |
| Bis-Vinyl Dimethicon / PPG-20 Crosspolymer und Isodecyl Neopentanoat | 20 | 2,5 | | 12,5 | | 10 |
| Dimethicon / PPG-20 Crosspolymer und Isododecan | | 7,5 | 6 | | 5 | |
| Glycerin | 7,5 | 5 | 4 | 3 | 5 | 2 |
| Ethanol | | | 2 | | 4 | 2 |
| Bis-Ethylhexyloxyphenol Methoxyphenyltriazin | 2 | 3 | 0,5 | 1,5 | 3 | 2 |
| Ethylhexylmethoxycinnamat | | | 7 | | 8 | 1 |
| Octylsalicylat | | | | | 5 | 3 |
| Homosalat | | | 3 | | | |
| Octocrylen | 5 | | | 4 | 4 | |
| Phenylbenzimidazol Sulfonsäure | 1 | | | 2 | | |
| Butyl Methoxydibenzoylmethan | 2 | | 3 | 4 | 1 | |
| 2,4,6-Tris-(biphenyl)-1,3,5-triazin | | 2 | | | 1 | |
| Diethylhexylbutamidotriazin | | | 2 | 1,5 | | 2 |
| Ethylhexyltriazin | | 1 | | 1,5 | | |
| Creatin | 0,5 | | | 0,5 | 0,2 | |
| Coenzym Q 10 | 0,1 | 0,02 | | | | |
| Vitamin E Acetat | 0,5 | | 0,3 | 0,3 | 0,8 | 0,5 |
| Na₂H₂EDTA | 0,1 | | | | | 0,5 |
| Parfüm | 0,2 | 0,3 | 0,4 | 0,5 | | |
| Parabene | 0,5 | 0,4 | 0,5 | 0,3 | 0,3 | 0,4 |
| Natriumhydroxid, Farbstoffe | q.s. | q.s. | q.s. | q.s. | q.s. | q.s. |
| Wasser | ad 100,0 | ad 100,0 | ad 100,0 | ad 100,0 | ad 100,0 | ad 100,0 |

| W/O-Emulsionen | 17 | 18 | 19 | 20 | 21 |
|---|---|---|---|---|---|
| Triglycerindiisostearat | 1,0 | 0,5 | 0,25 | 2,0 | 3,0 |
| Diglycerindipolyhydroxystearat | 1,0 | 1,5 | 1,75 | 3,0 | 2,0 |
| Paraffinöl | 12,5 | 10,0 | 8,0 | 5,0 | 11,5 |
| Vaseline | 8,0 | 6,0 | 5,0 | 12,0 | 2,5 |
| Hydrierte Kokosglyceride | 2,0 | 1,0 | 2,5 | 5,0 | 0,25 |
| Decyloleat | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| Hydriertes Rizinusöl | 0,5 | 0,75 | 1,0 | 2,5 | 5,0 |
| Dimethicon / PPG-20 Crosspolymer und Isododecan | --- | 12,5 | 2,5 | --- | --- |
| Bis-Vinyl Dimethicon / PPG-20 Crosspolymer und Isodecyl Neopentanoat | 7,5 | --- | --- | 5 | 20 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Natriumcitrat | 0,2 | 0.05 | 0.4 | 0.3 | 2,0 |
| Parfum | q,s, | q,s, | q,s, | q,s, | q,s, |
| Ethanol | 2,0 | --- | 5,0 | --- | --- |
| Capryl-/Caprinsäuretriglycerid | 2,0 | 2,5 | 3,0 | 5,0 | 0,5 |
| Kaliumsorbat | 0,04 | 0,15 | 0,05 | 0,03 | 0,4 |
| Benzylalkohol . | 0,3 | 0,4 | 0,25 | 0,15 | --- |
| Wasser | ad 100 | ad 100 | ad 100 | ad 100 | ad 100 |

## Patentansprüche

1. Verwendung von ein oder mehreren ABC-Siloxanelastomere, erhältlich aus der Reaktion von
A-Organohydrogensiloxanen mit mindestens zwei SiH tragenden Cyclosiloxanringen pro Molekül, mit
B-Stoffen oder Mischungen mit mindestens zwei aliphatischen ungesättigten Gruppen oder Hydrocarbongruppen pro Molekül und einem
C-Hydrosilylationskatalysator zur Verbesserung der mikrobiologischen Stabilität kosmetischer Zubereitungen.

2. Verwendung nach Anspruch 1, **dadurch gekennzeichnet, dass** als ABC-Siloxanelastomere Dimethicone/PPG-20 Crosspolymer, Dimethicone/ Bis-Isobutyl PPG-20 Crosspolymer und/oder Bis-Vinyl Dimethicone/PPG-20 Crosspolymer gewählt werden.

3. Verwendung nach Anspruch 1, **dadurch gekennzeichnet, dass** den Zubereitungen ein oder mehrere polare Öle und/oder Wachse zugesetzt werden.

4. Verwendung nach Anspruch 1, 2 oder 3, **dadurch gekennzeichnet, dass** die ABC-Siloxanelastomere zu einem Anteil von mehr als 1,5 Gew.%, bezogen auf die Gesamtmasse der Zubereitung, in der Zubereitung enthalten sind.
